(19) Europäisches Patentamt
European Patent Office
Office européen des brevets

(11) **EP 1 078 612 B1**

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(51) Int Cl.:
***A61F 2/38*** *(2006.01)*

(21) Anmeldenummer: **00810598.3**

(22) Anmeldetag: **10.07.2000**

(54) **Künstliches Kniegelenk**

Knee prosthesis

Prothèse de genou

(84) Benannte Vertragsstaaten:
**AT BE CH CY DE DK ES FI FR GB GR IE IT LI LU MC NL PT SE**

(30) Priorität: **10.08.1999 EP 99810719**

(43) Veröffentlichungstag der Anmeldung:
**28.02.2001 Patentblatt 2001/09**

(73) Patentinhaber: **Zimmer GmbH**
**8404 Winterthur (CH)**

(72) Erfinder: **Leclercq, Vincent**
**8404 Winterthur (CH)**

(74) Vertreter: **Manitz, Finsterwald & Partner GbR**
**Postfach 31 02 20**
**80102 München (DE)**

(56) Entgegenhaltungen:
**EP-A- 0 529 408**

EP 1 078 612 B1

**Beschreibung**

Die Erfindung handelt von einem künstlichen Kniegelenk mit einem Meniskusteil, welches auf einer Tibiaplattform verschiebbar gelagert ist, und mit einem relativ zur Tibiaplattform drehbar gelagerten Lenker, der in eine Führung des Meniskusteils eingreift, wobei der Lenker und das Meniskusteil seitliche Führungsflächen zur Führung des Meniskusteils aufweisen.

In der EP-A-0 529 408 wird ein Meniskusteil auf einer Tibiaplattform gelagert und ist durch einen drehbaren Lenker geführt. Dabei entsteht eine geradlinige Längsführung durch parallele Führungsflächen von Lenker und Meniskusteil, der die Drehung des Lenkers überlagert werden kann. Es ist üblich, die sich berührenden Führungsflächen in unterschiedlichen Werkstoffen wie zum Beispiel Polyethylen und Metall herzustellen, um günstige Gleitpaarungen herzustellen. Bei Kunststoffen wie Polyethylen muss man darauf achten, dass die spezifischen Druckkräfte nicht zu gross werden, um Kaltfluss und Abrieb zu verhindern.

Bei Knieprothesen der oben erwähnten Art treten die grössten zu übertragenden Seitenkräfte zwischen Lenker und Meniskusteil in der gestreckten Position auf, in der das Meniskusteil eine vordere Lage auf der Tibiaplattform einnimmt. Nur in dieser annähernd gestreckten Position kann ein Prothesenträger analog zum natürlichen Gelenk grosse Lasten tragen, sich seitlich wegstemmen oder Schläge erhalten, die nicht durch Muskeln und Bänder abgefedert werden. Gleichzeitig ist das Meniskusteil in seiner vordersten Position am weitesten vom Drehpunkt des Lenkers entfernt, so dass für eine seitliche Kraftübertragung ungünstige Verhältnisse entstehen können.

Aufgabe der Erfindung ist es, Führungssysteme von Lenker und Meniskus aufzuzeigen, bei denen überhöhte Flächenpressungen unter Seitenkräften vermieden werden. Diese Aufgabe wird gemäss Anspruch 1 dadurch gelöst, dass die seitlichen Führungsflächen des Lenkers und die zugehörigen Führungsflächen des Meniskusteils unterschiedliche Krümmungsradien $R_1$, $R_2$ aufweisen, die grösser als 10 cm sind, wobei die Summe der Reziproke $\frac{1}{R_1} + \frac{1}{R_2}$ kleiner als 0,2 cm$^{-1}$ ist. Vorteilhafte Weiterbildungen der Erfindung ergeben sich aus den abhängigen Ansprüchen 2 bis 10.

Ein Vorteil der Erfindung besteht darin, dass konstruktiv der Drehpunkt M für den Lenker relativ weit gegen posterior in die Richtung der am natürlichen Gelenk vorhandenen Kreuzbänder gelegt werden kann, ohne dass deswegen unzulässige Spannungsspitzen bei Seitenkräften auftreten.

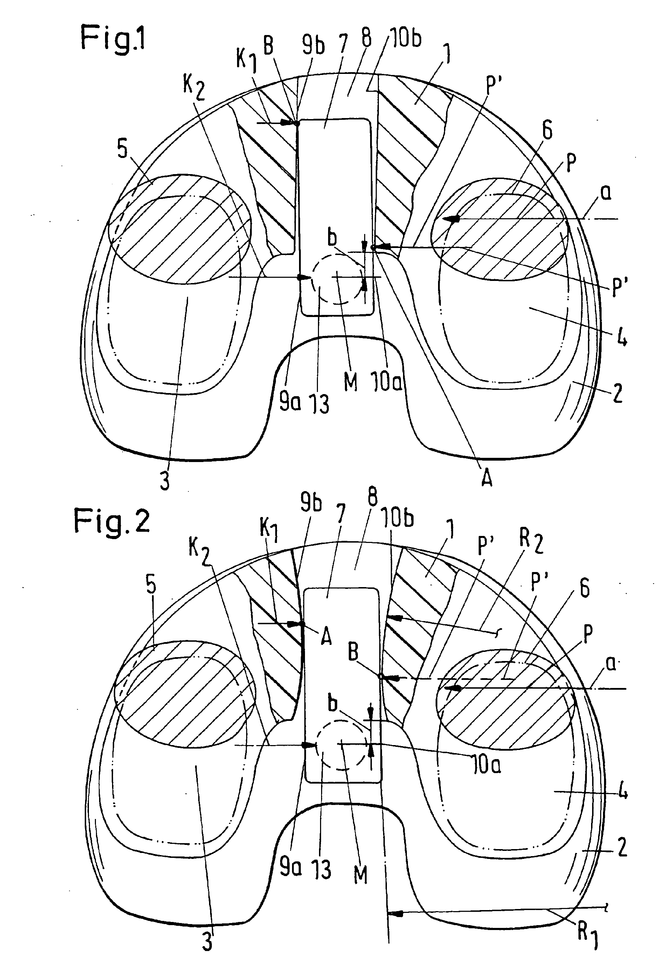

Dadurch dass Kontaktpunkte A, B zwischen Lenker und Meniskusteil in Führungsrichtung mit Krümmungsradien von grösser als 10 cm behaftet sind, ergeben alle möglichen Krümmungskombinationen einen günstigeren Wert als eine Konstellation, wie sie beispielsweise in Figur 1 als Stand der Technik gezeigt ist.

Am Beispiel von Kugelflächen mit Kugeldurchmessern $D_1$, $D_2$ lässt sich der Einfluss der Krümmung zeigen. Wenn an einem Auflagepunkt nur der Durchmesser der Kugelfläche verändert wird, ist die maximale Flächenpressung

a) im Fall "Kugel mit Durchmesser D auf ebene Fläche" proportional zu

$$\sqrt[3]{\frac{1}{D^2}}$$

b) im Fall "Kugel $D_2$ gegen Kugel $D_1$" proportional zu

$$\sqrt[3]{\frac{1}{\left(\frac{D_1 \cdot D_2}{D_1 + D_2}\right)^2}}$$

c) im Fall "Kugel $D_2$ gegen grössere Kugelschale $D_1$" proportional zu

$$\sqrt[3]{\frac{1}{\left(\frac{D_1 \cdot D_2}{D_1 - D_2}\right)^2}}$$

Wenn man auf dieser Basis einen theoretischen Vergleich durchführt, ergibt sich für eine Kugel, deren Durchmesser mit 1 cm etwa der Breite eines Lenkers entspricht, im Fall a) ein Wert von 1, dem mit einer Kugel von 20 cm ein Wert von 0,13 und im Fall c) mit einer Kugel von 20 cm gegen eine Schale von 200 cm Durchmesser ein Wert von 0,126 gegenüber steht, was einer Verbesserung um einen Faktor 7 entsprechen würde. Selbst im Fall b) würden zwei Kugelflächen mit Durchmesser 20 cm und 200 cm noch einen Wert von 0,14 ergeben, was einer Verbesserung um einen Faktor von 6,9 entspricht.

Aus der Sicht der Flächenpressung sind möglichst grosse Krümmungsradien an den Kontaktpunkten von Vorteil. Praktisch sind jedoch Grenzen gesetzt, indem die Kontaktpunkte im Bereich der sich überdeckenden Führungsflächen liegen müssen. Je grösser die Krümmungsradien sind, desto grösser ist der Einfluss der Herstelltoleranzen und des Spiels zwischen Lenker und Meniskus darauf, dass sich Kontaktpunkte A, B noch innerhalb der sich überdeckenden Führungsflächen mit vorgegebenen Krümmungsradien befinden. Aus herstelltechnischen Gründen sind daher die Lösungen von Vorteil, bei denen sich die Führungsflächen einfach und in engen Toleranzen herstellen lassen, da das Spiel von der Genauigkeit abhängt, mit der die Führungsflächen des Meniskus in einem vorgegebenen Abstand zueinander und die Führungsflächen des Lenkers in einem vorgegebenen Abstand zueinander liegen. Bei hoher Genauigkeit der vorgegebenen Abstände kann der kleinere der Krümmungsradien $R_1$, $R_2$ Beträge grösser als 20 cm annehmen und die Summe der Reziprokwerte der Krümmungsradien $\frac{1}{R_1} + \frac{1}{R_2}$ kleiner als 0,1 cm$^{-1}$ sein. Bei sehr hoher Genauigkeit der vorgegebenen Abstände kann der kleinere der Krümmungsradien $R_1$, $R_2$ Beträge grösser als 30 cm annehmen und die Summe der Reziprokwerte der Krümmungsradien $\frac{1}{R_1} + \frac{1}{R_2}$ kleiner als 0,067 cm$^{-1}$ sein. Bei dieser Betrachtung sind ebenen Führungsflächen unendlich grossen Krümmungsradien zugeordnet.

Eine herstelltechnisch einfache Ausführungsform ergibt sich mit einem Meniskusteil, dessen Führungsflächen gerade und parallel zueinander verlaufen und einem Lenker mit konvexen Führungsflächen. Statt gerade können die Führungsflächen des Meniskusteils auch leicht konkav verlaufen mit einem wesentlich grösseren Krümmungsradius als dem des Lenkers.

Eine weitere Ausführungsform, die herstelltechnisch als schwieriger eingestuft wird, ergibt sich mit einem Lenker, dessen Führungsflächen gerade und parallel zueinander verlaufen und einem Meniskusteil mit konvexen Führungsflächen. Statt gerade können die Führungsflächen des Lenkers auch leicht konkav verlaufen mit einem wesentlich grösseren Krümmungsradius als dem des Meniskusteils.

Im folgenden wird die Erfindung anhand von Ausführungsbeispielen beschrieben. Es zeigen:

Fig. 1: schematisch aus dem Stand der Technik eine Draufsicht auf eine Tibiaplattform mit Lenker und Meniskus.

Fig.2 schematisch eine Draufsicht auf eine erfindungsgemässe Anordnung mit konvexen Führungsflächen am Lenker und mit konkaven Führungsflächen am Meniskusteil.

Fig.3 schematisch eine Draufsicht auf eine Anordnung mit zwei konvexen Lenkern, die unterschiedlich grosse Krümmungsradien aufweisen, wobei das Meniskusteil sich in der vorderen, d.h. anterioren Stellung befindet und ein gleiches Spiel zwischen den Führungsflächen besteht.

Fig.4 schematisch die Anordnung von Fig.3 mit dem kleineren Krümmungsradius $R_{11}$ der konvexen Führungsflächen des Lenkers.

Fig.5 schematisch die Anordnung von Fig.3 mit dem grösseren Krümmungsradius $R_{12}$ der konvexen Führungsflächen des Lenkers.

Fig.6 schematisch eine Anordnung mit konvexen Führungsflächen des Lenkers und mit konkaven Führungsflächen des Meniskusteils, und

Fig.7 schematisch eine Anordnung mit konvexen Führungsflächen des Lenkers und mit konvexen Führungsflächen des Meniskusteils.

In den Beispielen sind Anordnungen an einem künstlichen Kniegelenk gezeigt mit einem Meniskusteil, welches auf einer Tibiaplattform verschiebbar gelagert ist, und mit einem auf der Tibiaplattform drehbar gelagerten Lenker, der in eine Führung des Meniskusteils eingreift, wobei der Lenker und das Meniskusteil seitliche Führungsflächen (9a, 10a; 9b, 10b) aufweisen. Um die Flächenpressung zwischen den Führungsflächen zu begrenzen, weisen die Führungsflächen unterschiedliche Krümmungsradien $R_1$, $R_2$ mit Beträgen grösser als 10 cm auf und ist die Summe der Reziproke der Krümmungsradien $\frac{1}{R_1} + \frac{1}{R_2}$ kleiner als 0,2 cm$^{-1}$.

An der Darstellung von Figur 1 soll zunächst die Problematik der Seitenkräfte an einer herkömmlichen Anordnung aufgezeigt werden. Ein Lenker 7 mit geraden parallelen Führungsflächen 9a, 10a ist mit einem Zapfen 13 auf einer Tibiaplattform 2 gelagert und um einen Drehpunkt M drehbar. Die nur im Schnitt erkennbaren Kondylen 5, 6 sind in Lagerschalen 3, 4 des Meniskusteils gelagert und erfahren eine Seitenkraft P in einer Wirkungslinie a, welche sie auf das Meniskusteil 1 übertragen, welches seinerseits diese Seitenkraft an den Lenker 7 weitergibt. Die ebenfalls geraden und parallelen Führungsflächen 9b, 10b wirken nun auf den Lenker, um einen Gleichgewichtszustand zu erreichen, wobei sich der Lenker im Rahmen des Spiels der Seitenführungen 9a, 9b, 10a, 10b verdrehen kann, bis an einem Kontaktpunkt A eine dorthin reduzierte Seitenkraft P' und an einem Kontaktpunkt B eine Reaktionskraft $K_1$ sowie am Zapfen 13 eine Reaktionskraft $K_2$ entstehen. Entsprechend den in den Berührungspunkten A und B vorhandenen Krümmungsradien der Führungsflächen ergeben sich Flächenpressungen, die je nach Materialkombination, z.B. Polyethylen gegen Metall, zu einem unerwünscht hohen Verschleiss führen können, wobei weniger eine Vergrösserung des Spiels sondern das Entstehen von Verschleisspartikeln und deren Verbleib im menschlichen Körper unerwünscht sind.

Die in Figur 1 gezeichnete Position des Meniskusteils 1 entspricht der vordersten anterioren Stellung auf der Tibiaplattform. In dieser Stellung ist der sich überdeckende Bereich der Führungsflächen 9a, 9b und der Führungsflächen 10a, 10b um einen Abstand b vom Drehpunkt M des Zapfens 13 gegen anterior verschoben und es entsteht ein Moment P' b, welches die Reaktionskraft $K_1$ im Punkt B bestimmt. Unglücklicherweise treten die grössten Seitenkräfte in der annähernd gestreckten Stellung des Kniegelenks auf, so dass in dieser Stellung grosse Seitenkräfte und kleine Krümmungsradien an den Kontaktpunkten A und B zusammenfallen. Erschwerend kommt hinzu, dass der Lenker 7 in dieser Position nicht soweit wie das Meniskusteil 1 gegen anterior vorstehen darf, damit in der posterioren Stellung des Meniskusteils keine grossen Schultern des Lenkers über das Meniskusteil gegen anterior vorstehen, die bei einer Drehung des Meniskusteils Gewebeteile verletzen könnten. In den nachfolgenden erfindungsgemässen Ausführungsbeispielen sind die gleichen Hinweiszeichen wie in Figur 1 verwendet. Sie unterscheiden sich bezüglich ihrer Geometrie durch die Gestaltung der Seitenführungen 9a, 9b und 10a, 10b.

Im Beispiel der Figur 2 ist das Meniskusteil 1 in seiner anterioren Stellung dargestellt. In einem Zahlenbeispiel berägt der Abstand b vom Beginn der Führungsflächen 9b, 10b zum Drehpunkt M des Lenkers 7 0,542 cm. Der kürzeste Abstand zwischen den Führungsflächen 9b, 10b des Meniskusteils beträgt 1,35 cm. Die Seitenführungen 9b, 10b des Meniskusteils 1 sind konvex gekrümmt und weisen einen Krümmungsradius $R_2$ auf. Die Seitenführungen 9a, 10a des Lenkers 7 sind leicht konkav gewölbt und weisen einen Krümmungsradius $R_1$ auf, der wesentlich grösser als der Krümmungsradius $R_2$ ist. Im Zahlenbeispiel betragen $R_2$ = 32,0 cm; $R_1$ = 100,0 cm und die Verbindungsgerade der Mittelpunkte für die Krümmungsradien $R_2$ stellt gleichzeitig Halbierende und Symmetrieachse für die Führungsflächen 9b, 10b des Meniskusteils 1 dar, während die Verbindungsgerade der Mittelpunkte für die Krümmungsradien $R_1$ gleichzeitig Halbierende und Symmetrieachse für die Führungsflächen 9a, 10a des Lenkers darstellt. Das Gesamtspiel ist so gross bemessen, dass während der Verschiebung des Meniskusteils gegen posterior kein Verklemmen stattfindet. Je grösser der Krümmungsradius $R_1$ ist, desto geringer wird die Gefahr eines Verklemmens. Und mit ebenen und parallelen Führungsflächen 9a, 10a, denen ein unendlich grosser Krümmungsradius $R_1$ entspricht, kann kein Verklemmen mehr eintreten, sodass das Spiel zwischen Lenker und Führung besonders klein gewählt werden kann. Die Führung 8 ist in den nachfolgenden Fällen als Nut ausgeführt. Sie könnte grundsätzlich auch als Langloch ausgeführt sein. Das Spiel zwischen Lenker und Führung bestimmt zusammen mit der Grösse der Krümmungsradien $R_1$, $R_2$ und der in Längsrichtung zu den zugehörigen Führungsflächen eingehaltenen Lage der Mittelpunkte zu $R_1$ und $R_2$ den Ort der Kontaktpunkte A und B innerhalb der Ueberdeckung der Führungsflächen 9a, 9b und 10a, 10b. Man hat auf diese Weise die Möglichkeit, die Lage der Kontaktpunkte A, B und grosse Krümmungsradien $R_1$, $R_2$ zu wählen, um zu geringen Flächenpressungen zu kommen. Die leichte Verdrehung des Lenkers 7 gegenüber dem Meniskusteil 1 hat eine geringe Querverschiebung des Meniskusteils auf der Tibiaplattform 2 zur Folge, die als nicht bedeutsam eingestuft wird. Im Zahlenbeispiel ergibt sich in der anterioren Stellung des Meniskusteils ein Abstand von 1,314 cm in Längsrichtung zwischen den Punkten A und B und eine Verdrehung des Lenkers 1 um 1,3°.

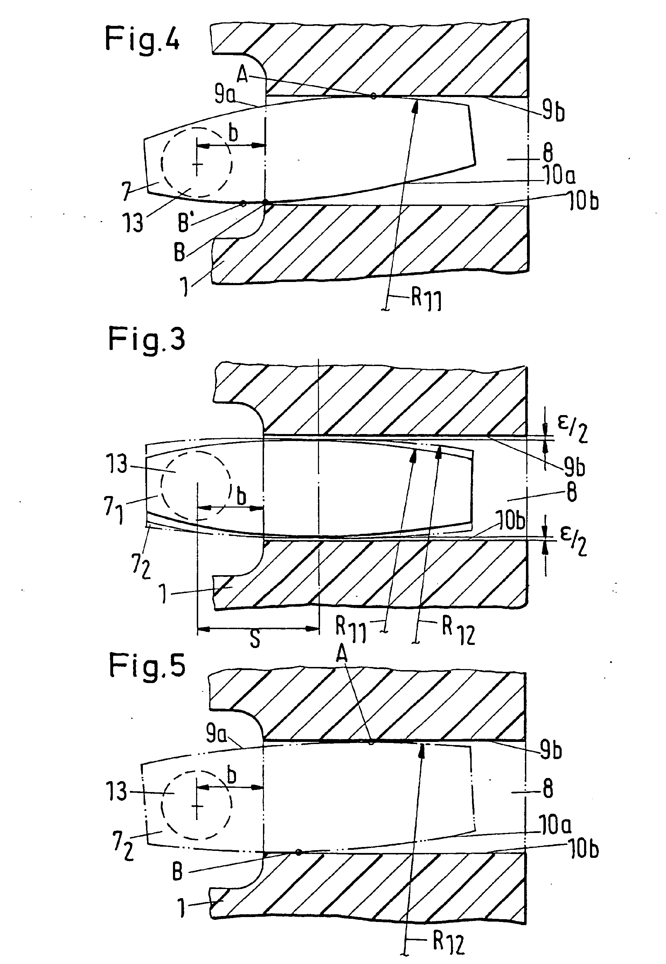

In den Figuren 3 und 5 ist eine weitere Anordnung mit einem Lenker $7_2$ mit konvexen Führungsflächen und mit einem Meniskusteil 1 mit geraden, parallelen Führungsflächen 9b, 10b gezeigt. Genau genommen sind in Figur 3 zwei Lenker $7_1$ und $7_2$ gezeigt, die - um ein Zahlenbeispiel zu geben - in neutraler Mittelstellung ein gleiches Gesamtspiel E = 0,02 cm gleichmässig zu den Führungsflächen 9b, 10b aufteilen. Die Lenker haben eine Länge von 2,75 cm und eine grösste Breite von 1,33 cm. Die Mittelpunkte der Krümmungsradien $R_1$ liegen auf der Halbierenden des Lenkers in Querrichtung. Diese Halbierende ist um den Betrag s = 0,778 cm vom Drehpunkt M des Lenkers gegen anterior versetzt. Die Führungsflächen 9b, 10b des Meniskusteils enden in einem Abstand b = 0,5 cm vor dem Drehpunkt M des Lenkers.

Der Lenker $7_1$ besitzt einen Krümmungsradius $R_{11}$ = 10,0 cm der Führungsflächen, der für das Spiel ε = 0,02 cm zu klein bemessen ist und, wie in Figur 4 angedeutet, dazu führt, dass die Kante vom Meniskusteil den Kontaktpunkt B bestimmt, während ein theoretischer Kontaktpunkt B' mit Krümmungsradius $R_{11}$ gegen eine Gerade ausserhalb der Ueberdeckung der Führungsflächen 10a, 10b von Lenker und Meniskusteil liegt. Eine Korrekturmöglichkeit, um hier die Schrägstellung des Lenkers von ursprünglichen 3° zu reduzieren und die Kontaktpunkte in die Ueberdeckung der Führungen zu bringen, besteht in der Reduktion des Spiels E. Eine andere Korrekturmöglichkeit ist die Vergrösserung des Krümmungsradius $R_{11}$.

In Figur 5 ist die Anordnung mit dem Lenker 72 gezeigt, dessen Führungsflächen 9a, 10a mit einem Krümmungsradius $R_{12}$ versehen sind, der bei sonst gleicher Anordnung zu einem Kontaktpunkt B innerhalb der Ueberdeckung der Führungsflächen 10a, 10b führt. Für das obige Zahlenbeispiel kann, bezogen auf Figur 3, eine solche Situation mit einem Radius $R_{12}$ = 32,0 cm, dessen Mittelpunkt um einen Betrag s = 0,778 cm vom Drehpunkt M des Zapfens gegen anterior verschoben ist, mit einem Lenker der Länge von 2,75 cm, mit einer Länge der Führungsflächen 9b, 10b des Meniskusteils von 1,8 cm, mit einem Abstand der Ueberdeckung vom Drehpunkt M von b = 0,5 cm und mit einem Spiel von E = 0,02 cm erreicht werden. Die Schrägstellung des Lenkers beträgt nur etwa 1,7°. Es versteht sich, dass die Herstelltoleranzen für die Führungsflächen 9a, 10a und 9b, 10b in diesem Spiel ε = 0,02 cm mit enthalten sein müssen, damit nicht eine Situation wie in Figur 4 eintritt.

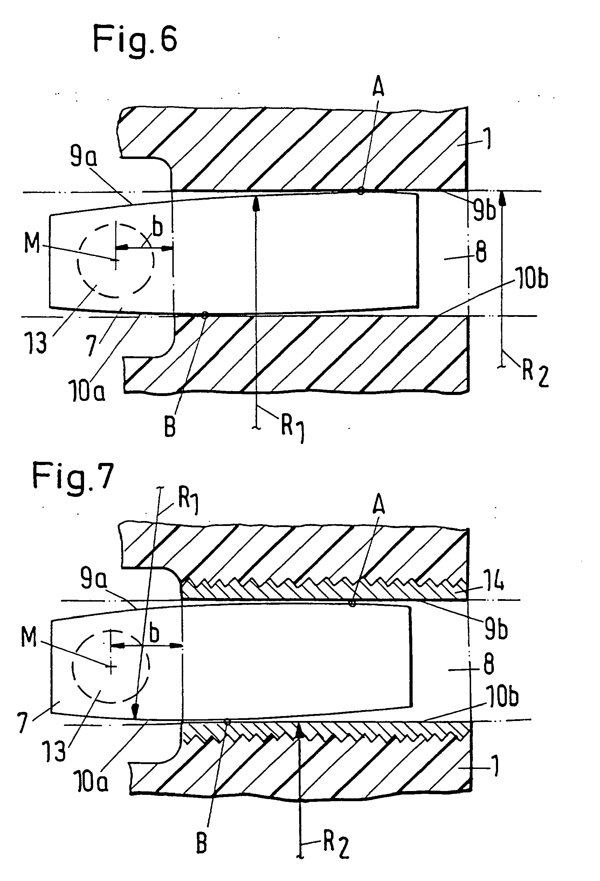

Im Beispiel von Figur 6 sind die Führungsflächen 9b, 10b des Meniskusteils im Vergleich zur Figur 5 nicht mehr parallel und gerade, sondern sehr wenig konkav gekrümmt. Die Mittelpunkte der Krümmungsradien $R_1$ des Meniskusteils liegen quer zu den Führungsflächen 9b, 10b auf deren Mittellinie in Querrichtung. Um bei den Zahlen des Beispiels zur Figur 5 zu bleiben, würde ein Krümmungsradius $R_2$ = 100,0 cm und ein engster Abstand von 1,35 cm zwischen den Führungsflächen 9b, 10b des Meniskusteils 1 am Beginn der Führungsflächen genügen, um Kontaktpunkte A und B innerhalb der Ueberdeckung zu erhalten und diese innerhalb der Ueberdeckung beizubehalten, bis sich deren Abstand b zum Drehpunkt M auf Null verkürzt hat.

Im Beispiel von Figur 7 sind die Führungsflächen 9b, 10b des Meniskusteils 1 im Vergleich zur Figur 5 nicht mehr parallel und gerade, sondern sehr wenig konvex gekrümmt. Auch hier ist es möglich, mit einem sehr grossen Krümmungsradius $R_2$ von beispielsweise 100,0 cm eine Verschiebung aus der anterioren Stellung um einen Betrag b vorzunehmen, ohne dass dieser Krümmungsradius $R_2$ auf dem Meniskusteil 1 vom Kontaktpunkt B oder A verlassen wird. Allerdings werden bei diesem letzten Ausführungsbeispiel besonders hohe Anforderungen an das einzuhaltende Spiel resp. an die Herstelltoleranzen gestellt. Bei einem Lenker des Zahlenbeispiels zu Figur 5 und einem kürzesten Abstand von 1,35 cm zwischen den Führungsflächen 9b, 10b an deren Halbierenden ergibt sich eine Schrägstellung des Lenkers in der anterioren Stellung des Meniskusteils von 1,8° und es entstehen Kontaktpunkte A, B innerhalb der Ueberdeckung der Führungsflächen 9a, 9b und 10a, 10b.

Um ganz allgemein die Herstelltoleranzen von einer Führungsnut 8 in einem Kunststoff wie zum Beispiel Polyethylen klein zu halten, kann es von Vorteil sein, diese Nut in einem Einsatzstück 14 aus Metall herzustellen und dieses im Meniskusteil 1, wie in Figur 7 gezeigt, zu verankern. Eine solche Nut hat den Vorteil, dass sie den Abstand ihrer Führungsflächen 9b, 10b weder durch Quellen noch durch Temperaturschwankungen wesentlich verändert.

Ein weiterer Aspekt ist die mehrfache Verwendung eines Lenkers $7_2$, wie er in Figur 5 gezeigt ist, für verschieden grosse Meniskusteile 1 und für verschieden grosse Tibiaplattformen 2, um einen Lenker für verschiedene Kniegrössen einsetzen zu können. Bei einem solchen Einsatz kann es sich ergeben, dass nicht alle Kombinationen mit unterschiedlichen Meniskusteilen in der anterioren Stellung einen Abstand b ihrer Führungen 9b, 10b zum Drehpunkt M des Lenkers erzeugen, welcher zum Verkanten des Lenkers führt. Trotzdem ist es für die Teilebewirtschaftung von Vorteil, beispielsweise mit einem "Einheitslenker" mit konvexen Führungsflächen 9a, 10a und mit geraden parallelen Führungsflächen 9b, 10b des Meniskusteils 1 auch die Fälle abzudecken, in denen kein Verkanten auftritt.

## Patentansprüche

1. Künstliches Kniegelenk mit einem Meniskusteil (1), welches auf einer Tibiaplattform (2) verschiebbar gelagert ist, und mit einem relativ zur Tibiaplattform (2) drehbar gelagerten Lenker (7), der in eine Führung (8) des Meniskusteils eingreift, wobei der Lenker und das Meniskusteil (1) seitliche Führungsflächen (9a, 10a; 9b, 10b) zur Führung des Meniskusteils (1) aufweisen,
**dadurch gekennzeichnet, dass** die seitlichen Führungsflächen (9a, 10a) des Lenkers (7) und die zugehörigen Führungsflächen (9b, 10b) des Meniskusteils (1) unterschiedliche Krümmungsradien ($R_1$; $R_2$) aufweisen, die grösser als 10 cm sind, wobei die Summe der Reziproke der Krümrnungsradien $\frac{1}{R_1} + \frac{1}{R_2}$ kleiner als 0,2 cm$^{-1}$ ist.

2. Künstliches Kniegelenk nach Anspruch 1, **dadurch gekennzeichnet, dass** die Krümmungsradien ($R_1$, $R_2$) grösser als 20 cm sind, wobei die Summe der Reziproke der Krümmungsradien $\frac{1}{R_1} + \frac{1}{R_2}$ kleiner als 0,1 cm$^{-1}$ ist.

3. Künstliches Kniegelenk nach Anspruch 1 oder 2, **dadurch gekennzeichnet, dass** die Krümmungsradien ($R_1$, $R_2$) grösser als 30 cm sind, wobei die Summe der Reziproke der Krümmungsradien $\frac{1}{R_1} + \frac{1}{R_2}$ kleiner als 0,067 cm$^{-1}$ ist.

4. Künstliches Kniegelenk nach einem der Ansprüche 1 bis 3, **dadurch gekennzeichnet, dass** die Führungsflächen (9a, 10a) des Lenkers (7) konvex und die Führungsflächen (9b, 10b) des Meniskus konkav oder geradlinig sind.

5. Künstliches Kniegelenk nach einem der Ansprüche 1 bis 3, **dadurch gekennzeichnet, dass** die Führungsflächen (9b, 10b) des Meniskus konvex und die Führungsflächen des Lenkers konkav oder geradlinig sind.

6. Künstliches Kniegelenk nach einem der Ansprüche 1 bis 5, **dadurch gekennzeichnet, dass** die Führungsflächen (9a, 10a) des Lenkers (7) metallisch und die Führungsflächen (9b, 10b) des Meniskus aus Kunststoff sind.

7. Künstliches Kniegelenk nach einem der Ansprüche 1 bis 5, **dadurch gekennzeichnet, dass** die Führungsflächen (9a, 9b; 10a, 10b) von Lenker und Meniskus metallisch sind.

**8.** Künstliches Kniegelenk nach einem der Ansprüche 1 bis 7, **dadurch gekennzeichnet, dass** der Drehpunkt M des Lenkers (7) in Längsrichtung von der Mitte des Lenkers betrachtet gegen posterior verschoben ist.

**9.** Künstliches Kniegelenk nach einem der Ansprüche 1 bis 8, **dadurch gekennzeichnet, dass** verschieden grosse Meniski, die sich auch durch die Länge ihrer Führungsflächen (9b, 10b) unterscheiden, mit dem gleichen Lenker (7) kombinierbar sind.

**10.** Künstliches Kniegelenk nach einem der Ansprüche 1 bis 9, **dadurch gekennzeichnet, dass** der Lenker einen Drehzapfen (13) aufweist, welcher in der Tibiaplattform gelagert ist.

**Claims**

**1.** An artificial knee joint having a meniscus part (1) which is displaceably journalled on a tibia platform (2), and having a guiding part (7) which is rotatably journalled relative to the tibia platform (2) and which engages into a guide (8) of the meniscus part, wherein the guiding part and the meniscus part (1) have lateral guiding surfaces (9a, 10a; 9b, 10b) for guiding the meniscus part (1), **characterized in that** the lateral guiding surfaces (9a, 10a) of the guiding part (7) and the associated guiding surfaces (9b, 10b) of the meniscus part (1) have different radii of curvature ($R_1$, $R_2$) which are greater than 10 cm, with the sum of the reciprocals of the radii of curvature $\dfrac{1}{R_1} + \dfrac{1}{R_2}$ being less than 0.2 cm$^{-1}$.

**2.** An artificial knee joint in accordance with claim 1, **characterized in that** the radii of curvature ($R_1$, $R_2$) are greater than 20 cm, with the sum of the reciprocals of the radii of curvature $\dfrac{1}{R_1} + \dfrac{1}{R_2}$ being less than 0.1 cm$^{-1}$.

**3.** An artificial knee joint in accordance with claim 1 or claim 2, **characterized in that** the radii of curvature ($R_1$, $R_2$) are greater than 30 cm, with the sum of the reciprocals of the radii of curvature $\dfrac{1}{R_1} + \dfrac{1}{R_2}$ being less than 0.067 cm$^{-1}$.

**4.** A artificial knee joint in accordance with any one of the claims 1 to 3, **characterized in that** the guiding surfaces (9a, 10) of the guiding part (7) are convex and the guiding surfaces (9b, 10b) of the meniscus are concave or rectilinear.

**5.** An artificial knee joint in accordance with any one of the claims 1 to 3, **characterized in that** the guiding surfaces (9b, 10b) of the meniscus are convex and the guiding surfaces of the guiding part are concave or rectilinear.

**6.** An artificial knee joint in accordance with any one of the claims 1 to 5, **characterized in that** the guiding surfaces (9a, 10a) of the guiding part (7) are metallic and the guiding surfaces (9b, 10b) of the meniscus are of plastic.

**7.** An artificial knee joint in accordance with any one of the claims 1 to 5, **characterized in that** the guiding surfaces (9a, 9b; 10a 10b) of the guiding part and the meniscus are metallic

**8.** An artificial knee joint in accordance with any one of the claims 1 to 7, **characterized in that** the point of rotation M of the guiding part (7) is displaced toward posterior 15 in a longitudinal direction when viewed from the middle of the guiding part.

**9.** An artificial knee joint in accordance with any one of the claims 1 to 8, **characterized in that** differently sized menisci, which also differ by the length of their guiding surfaces (9b, 10b), can be combined with the same guiding part (7).

**10.** An artificial knee joint in accordance with any one of the claims 1 to 9, **characterized in that** the guiding part has a rotation pin (13) which is journalled in the tibia platform.

**Revendications**

**1.** Prothèse du genou, comprenant une partie de ménisque (1) qui est posée de manière déplaçable sur une plate-forme tibiale (2), et comprenant un bras (7) monté en rotation par rapport à la plate-forme tibiale (2), qui s'engage dans un guidage (8) de la partie de ménisque, le bras et la partie de ménisque (1) présentant des surfaces de guidage latérales (9a, 10a ; 9b, 10b) pour le guidage de la partie de ménisque (1),
**caractérisée en ce que** les surfaces de guidage latérales (9a, 10a) du bras (7) et les surfaces de guidage associées (9b, 10b) de la partie de ménisque (1) présentent des rayons de courbure ($R_1$ ; $R_2$) qui sont supérieurs à 10 cm, et la somme des inverses des rayons de courbure ($1/R_1 + 1/R_2$) est inférieure à 0,2 cm$^{-1}$.

**2.** Prothèse du genou selon la revendication 1, **carac-**

**térisée en ce que** les rayons de courbure ($R_1$, $R_2$) sont supérieurs à 20 cm, et la somme des inverses des rayons de courbure ($1/R_1 + 1/R_2$) est inférieure à 0,1 cm$^{-1}$.

3. Prothèse du genou selon la revendication 1 ou 2, **caractérisée en ce que** les rayons de courbure ($R_1$, $R_2$) sont supérieurs à 30 cm, et la somme des inverses des rayons de courbure ($1/R_1 + 1/R_2$) est inférieure à 0,067 cm$^{-1}$.

4. Prothèse du genou selon l'une des revendications 1 à 3, **caractérisée en ce que** les surfaces de guidage (9a, 10a) du bras (7) sont convexes, et les surfaces de guidage (9b, 10b) du ménisque sont concaves ou rectilignes.

5. Prothèse du genou selon l'une des revendications 1 à 3, **caractérisée en ce que** les surfaces de guidage (9b, 10b) du ménisque sont convexes, et les surfaces de guidage du bras sont concaves ou rectilignes.

6. Prothèse du genou selon l'une des revendications 1 à 5, **caractérisée en ce que** les surfaces de guidage (9a, 10a) du bras (7) sont métalliques et les surfaces de guidage (9b, 10b) du ménisque sont en matière plastique.

7. Prothèse du genou selon l'une des revendications 1 à 5, **caractérisée en ce que** les surfaces de guidage (9a, 9b ; 10a, 10b) du bras et du ménisque sont métalliques.

8. Prothèse du genou selon l'une des revendications 1 à 7, **caractérisée en ce que** le centre de rotation M du bras (7), considéré en direction longitudinale depuis le milieu du bras, est déporté en direction postérieure.

9. Prothèse du genou selon l'une des revendications 1 à 8, **caractérisée en ce que** des ménisques de tailles différentes, qui diffèrent également par la longueur de leurs surfaces de guidage (9b, 10b) peuvent être combinés avec le même bras (7).

10. Prothèse du genou selon l'une des revendications 1 à 9, **caractérisée en ce que** le bras comprend un tenon de rotation (13), lequel est monté dans la plate-forme tibiale.

Fig.1

Fig.2

Fig.4

Fig.3

Fig.5

## Fig.6

## Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0529408 A